# EUROPEAN PATENT APPLICATION

(11) **EP 4 104 895 A1**
(43) Date of publication of application: **21.12.2022**
(21) Application number: 21188159.4
(22) Date of filing: 28.07.2021
(51) Int. Cl.: A61N 1/04, A61B 90/98, A61N 1/32

(54) **INTELLIGENT BIOMIMETIC BIODEVICE AND USE THEREOF**

(30) Priority: 14.06.2021 PT 2021117287
(71) Applicant: Instituto Politécnico De Leiria, 2411-901 Leiria (PT)
(72) Inventor: FERNANDES PATRÍCIO, Tatiana Marisa, 2425-900 Leiria (PT); FERNANDES ALVES, Nuno Manuel, 2430-441 Leiria (PT); DA SILVA CARREIRA, Pedro José, 2405-034 Leiria (PT); MENDES GAMEIRO, Fábrio André, 3100-670 Leiria (PT); DOS SANTOS MATEUS, Artur Jorge, 2400-187 Leiria (PT)
(74) Representative: Monteiro Alves, Inês

(57) **Abstract**

The present invention refers to an intelligent biomimetic biodevice (18), which is configured to communicate and activate, by means of electric and magnetic stimulation, particles incorporated in three-dimensional models (19) which present properties that vary according to the intended use.

The referred biodevice (18) is used in the prevention and in the treatment of diseases associated with the skeletal system, by means of the activation of the particles and release of therapeutic agents. Further, it is useful in the monitoring of the evolution of the regeneration process, by means of the monitoring of specific indicators (such as, strength, pH, oxygen, temperature and/or biomarkers).

The invention appears as a therapeutic alternative for the treatment of diseases associated to bone degeneration and, further, solves the problems related to the need for real time monitoring and/or control of the chosen treatment for the disease to be treated.

## Description

### TECHNICAL DOMAIN

The invention refers to an intelligent biomimetic biodevice which is capable of communicating and activating particles incorporated in three-dimensional models by means of electric and magnetic stimulations, with the purpose of preventing and treating diseases of the skeletal system, as well as monitoring the treatment of the disease in question.

### STATE OF THE ART

The bone defects cause a reduction in the quality of life and can result from countless factors, such as traumas, degenerative diseases, oncological diseases, natural aging process or obesity.

Several commercially available devices help to tackle the diseases associated to bone degeneration, however, many times, they cause adverse effects which can extend the treatment, leading to an increase in costs to the health system in question.

This situation can be minimized by means of the use of a bone substitute with biomimetic properties which follows the regeneration process, as well as a method that allows monitoring the treatment, thus controlling the possible triggering of an inflammatory process and/or rejection of the referred substitute, which would lead to a new surgical intervention and adverse effects for the patients.

The implementation of monitoring in the biodevice allows carrying out a customized treatment, since the typology of the disease, the age, the gender, are some of the factors that influence the regenerative process.

In view of the above-mentioned context, studies have been developed with the purpose of elaborating alternative technologies which can be useful in the treatment of diseases associated to bone degeneration, as well as the need for real time monitoring and/or control of the treatment chosen for the disease to be treated.

Implantable sensors for the detection of signals which indicate changes in the bone tissue are already known in the state of the art.

The technology described in US 2007/0238992 A1 uses acoustic or impedance sensors to follow the integrity of the bone tissue after a surgical procedure and, thus, help the doctor in determining the most suitable treatment and physical therapy.

In prior art document US 8,016,859 B2, pressure sensors are implanted in the spinal intradiscal space, and generate signals, in case a compressive force above a predetermined threshold is detected.

Prior art documents CN 101287408 A and EP 2 510 873 B1, in turn, describe ultrasonic sensors that are useful in the determination of biometric parameters, such as tension force and relative position.

The integration between sensors and medical devices is also known to the art, with the purpose of helping medical treatment.

In document CN 105943145 B, a hydraulic or mechanical implantable device, which will be applied to a bone, is adjusted from the detection of the physical parameters of the patient or the functional parameters of the device itself, by means of sensors. In US 2013/0138017 A1, a bone distraction device applies guided incremental forces to adjust the bone distraction rate based on the response received by sensors.

Patent US 7,932,825 B2 describes the integration of a sensor in an interactive medical implant, which allows the doctor to have access to the database of the patient, including his medical history, the treatment to which the patient was submitted, and history of the implanted device.

In a different manner, the present invention provides an intelligent biomimetic biodevice which is capable of communicating and activating, by means of electric and magnetic stimulation, particles incorporated in three-dimensional models, promoting a customized treatment.

During a disease or bone tissue regeneration process, several cell activities are triggered. These are an important tool which can be analyzed in real time by means of the device.

The present invention proposes the monitoring of the mechanical properties, the metabolism, and the levels of vascular perfusion of the tissues to treat the disease and/or evaluate the treatment chosen.

Although magnetic waves have already been used to act in the functioning of an implantable device, such as described in US 6,839,596 B and US 7,024,249 B2, the technology proposed in the present invention differs from the referred documents since it provides a monitoring in real time of the disease to be treated by means of the efficient detection of biomarkers, as well as the dynamic control of the used therapy.

The particles used in the present invention are also biomimetic and magnetic and further comprise antimicrobial and therapeutic agents that are suitable for the intended use, which the use may be for the prevention and the treatment of diseases associated to the skeletal system or the monitoring of the treatment to prove the efficacy thereof.

The article *"Bio-inspired polymeric iron-doped hydroxyapatite microspheres as a tunable carrier of rhBMP-2*" shows the capacity of superparamagnetic microspheres having a composition similar to bone to capture and distribute recombinant human bone morphogenetic protein (rhBMP-2) in therapeutically relevant doses, apart from their osteogenic properties, which can be observed by the presence of three donors of human mesenchymal cells.

In the article "*Superparamagnetic hybrid microspheres affecting osteoblasts behavior*"*,* the study performed shows that, in the presence of calcium and iron ions originating from hybrid microspheres in a culture which mimics the physiological environment, the osteoblasts present viability and cell differentiation.

The article "*New bioactive bone-like microspheres with intrinsic magnetic properties obtained by bio-inspired mineralization process*" presents the process for obtaining hybrid magnetic particles by biomineralization followed by hydrothermal stabilization.

In this context, the present invention emerges as a therapeutic alternative for the treatment of diseases associated to bone degeneration.

### PROBLEMS OF THE PRIOR ART

Most of the studies and publications relative to the biodevices teach implantable devices used for the detection of biometric signs or target analytes. In the case of the analytes, the biodevice binds or interacts with the same and a detector element transforms the signal resulting from the interaction in another type of signal which can be measured and quantified.

In this manner, in view of the need to develop new therapeutic alternatives for treating diseases associated to bone degeneration and, at the same time, solve the problems referring to the need for monitoring and/or control in real time of the chosen treatment, the present invention provides a biodevice which is capable of communicating and activating particles incorporated in three-dimensional models by means of electric and magnetic stimulation.

The properties of flexibility, thermal stability, permeability, water repellence, transparency, and comfort, associated to the fact that they are easily and painlessly removable and present good adhesion to the skin, make the biodevice of the present invention the most indicated choice in the cases where a monitoring of the disease and of the regenerative process in a continuous and customized manner are desired.

### SUMMARY OF THE INVENTION

The present invention refers, in a first aspect, to an intelligent biomimetic biodevice, which is configured to communicate and activate, by means of electric and magnetic stimulation, particles incorporated in three-dimensional models, which comprises:
a) an intelligent biomimetic surface comprising transducers, sensors and electric stimulation electrode;
b) a magnetic stimulation subsystem comprising at least two magnetic stimulation coils;
c) a signal amplification subsystem, comprising a sensor signal amplification module, an RFID communication module and a WI-FI / BLUETOOTH communication module; and
d) a functional electronic subsystem comprising an intelligent biomimetic surface transducer processing module; a power module and a central processing unit.

The present invention further describes, in a second aspect, the use of the referred biodevice in the prevention and in the treatment of diseases associated to the skeletal system.

Additionally, the biodevice is used in the monitoring of the evolution of the regeneration process, by means of the monitoring of specific indicators (such as strength, pH, oxygen, temperature and/or biomarkers).

### SOLUTION OF THE PROBLEM

The present invention solves countless problems of the state of the art related to the use of medical devices for the treatment of diseases associated to bone degeneration.

A first point refers to the inflammation and rejection processes resulting from the use of bone substitutes, which, due to their characteristics, do not take into account the specificities of the patient to offer a personalized treatment. In the present invention, both the biodevice and the particles mimics the native tissues.

The biodevice is placed on the skin and presents a surface which facilitates the communication with the particles, which are incorporated in different three-dimensional models and, therefore, facilitate the method of implantation and improve the vascularization.

Their properties of flexibility, thermal stability, permeability, water repellence, transparency, and comfort, associated to the fact that they are easily and painlessly removed and present good adhesion to the skin, make the biodevice of the present invention the most indicated choice in the cases where a monitoring of the disease and of the regenerative process in a continuous and customized manner are intended.

Due to the countless properties, the invention contributes with an efficient and innovative alternative for the treatment of bone degeneration by means of the activation of the particles and the release of the therapeutic agents, as well as the monitoring of the evolution of the regeneration process in real time of the specific indicators selected, such as strength, pH, oxygen, temperature and/or biomarkers.

### ADVANTAGEOUS EFFECTS OF THE INVENTION

The main advantage of the intelligent biomimetic biodevice described in the present invention is associated to the capacity of interacting, by means of electric and magnetic stimulation, with the particles incorporated in three-dimensional models (such as injectable models, hydrogels and/or scaffolds), thus initiating the cellular activity and activating the referred three-dimensional model in a symbiotic manner.

From this interaction, the referred particles are activated in accordance with the desired purpose, that is, by the release of therapeutic agents to prevent or treat bone degeneration or, further, monitor and/or control the treatment in real time and in a customized manner by means of the detection of specific indicators selected, such as strength, pH, oxygen, temperature and/or biomarkers, which demonstrate the evolution of the regeneration process or the appearance of an inflammatory process. Thus, the biodevice will allow adjusting the electric or magnetic stimulation parameters to improve the therapy.

### BRIEF DESCRIPTION OF THE FIGURES

With the purpose of providing an understanding of the principles according to the embodiments of the present invention, reference will be made to the embodiments illustrated in the figures and to the terminology used to describe them.

It must further be understood that there is no intention of limiting the scope of the present invention to the content of the figures and that modifications of the inventive characteristics shown herein, as well as any additional applications of the principles and embodiments of the invention shown, which would occur normally to a person skilled in the art having this description in hands, are considered as being within the scope of the claimed invention.

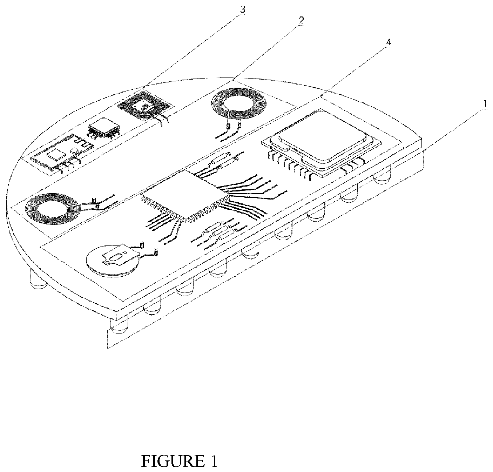

Figure 1 - illustrates a top perspective view of the intelligent biomimetic biodevice of the present invention and the different comprised subsystems.

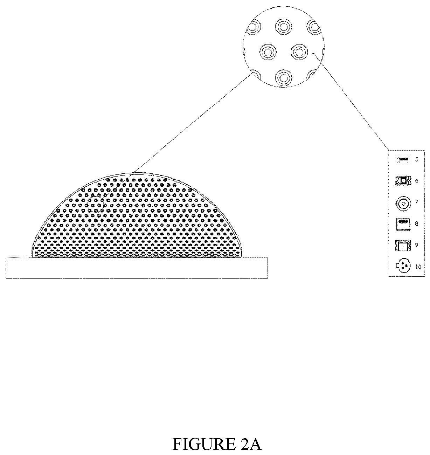

Figure 2A - illustrates a lower view of the intelligent biomimetic biodevice of the present invention, namely, the intelligent biomimetic surface, the sensors thereof and the electric stimulation electrode.

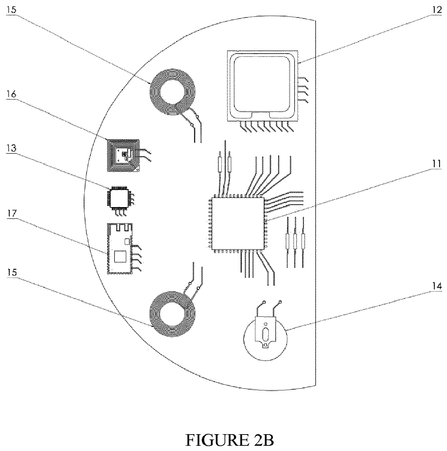

Figure 2B - illustrates a lower view of the intelligent biomimetic biodevice of the present invention, namely the different components that constitute the magnetic stimulation subsystems, of signal amplification and functional electronics.

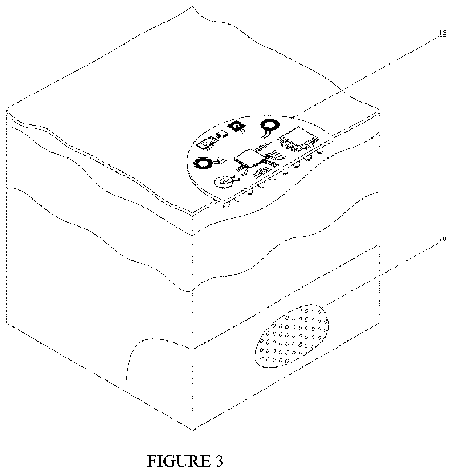

Figure 3 - illustrates the biodevice of this invention incorporated in the skin and the three-dimensional model with which it communicates and activates, incorporated in the bone.

### DESCRIPTION OF THE EMBODIMENTS

The present invention refers to an intelligent biomimetic biodevice (18), which is configured to communicate and activate, by means of electric and magnetic stimulation, particles incorporated in three-dimensional models (19).

The particles are also biomimetic and magnetic and further comprise antimicrobial and therapeutic agents, which properties vary according to the intended use.

The intelligent biomimetic biodevice (18) of the invention comprises:
a) an intelligent biomimetic surface (1) comprising transducers, sensors (5, 6, 7, 8, 9) and the electric stimulation electrode (10);
b) a magnetic stimulation subsystem (2) comprising at least two magnetic stimulation coils (15);
c) a signal amplification subsystem (3) comprising a signal amplification module (13) of the sensors (5, 6, 7, 8, 9), an RFID communication module (16) and a WI-FI / BLUETOOTH communication module (17); and
d) a functional electronic subsystem (4) comprising a transducer processing module (11) of the intelligent biomimetic surface (1); a power module (14) and a central processing unit (12).

The intelligent biomimetic biodevice (18) of the present invention is constructed from biocompatible materials, permeable and flexible, by means of additive manufacturing technologies and/or inkjet printing.

Since the skin is susceptible to factors that are external to the body (such as the body movements), the intelligent biomimetic biodevice (18) of the invention presents a *Young* modulus with tension varying from 4.6 MPa to 20 MPa, being suitable to the properties of the skin.

In the embodiment of the present invention, the intelligent biomimetic surface (1) is responsible for recognizing the analytes and which is comprised by natural or synthetic biocompatible materials.

The referred natural biocompatible materials are selected from one or more from the group consisting in collagen, gelatin, recombinant peptides, among others.

The referred synthetic biocompatible materials are selected from one or more of the group consisting in polycaprolactone, polylactic acid, poly(lactic-co-glycolic acid), among others.

Since it must adhere to the skin and communicate with the particles of the three-dimensional model (19), the material must allow the development of a geometry that presents low rugosity, thermal stability, and elasticity.

In this sense, the intelligent biomimetic surface (1) presents a microneedle biomimetic geometry and/or a biomimetic geometry in the shape of membranes or suction pads, which mimics the surface of the octopus tentacles or the hands of reptiles, to allow the adequate collection of information originating from the bone tissue. This further comprises enzymes or antibodies which identify the analytes and allow the collection of information from the implant location.

The elasticity is suitable to the skin properties and the intelligent biomimetic surface (1) detects the information of the injured tissue that can demonstrate the evolution of the bone regeneration process and analyze the inflammatory process.

In one preferred embodiment of the present invention, the enzyme is a metalloproteinase enzyme, or the antibodies are selected from one or more of osteonectin, osteocalcin, alkaline phosphatase, autophagy marker (LC3B) or interleukin-6 (IL-6), among others.

Some analytes originating from the bone tissue which may be used as treatment indicators are the selected proteins, such as collagen, alkaline phosphatase, osteocalcin, and calcium phosphate.

As inflammation indicators, there are observed the expression of IL-6, the pH, the temperature, the oxygenation, and the strength.

The intelligent biomimetic surface (1) further translates the information collected in signals, by means of sensors (5, 6, 7, 8, 9) which can be selected from one or more from the group consisting in electrochemical sensors (5, 6), piezoresistive (piezoelectric or capacitive) (7), electric field sensors (8), and sensors that are based on the principle of photoplethysmography (9), or a combination thereof.

The electrochemical sensors (5, 6) present potentiometry and/or amperometry mechanisms and/or voltammetry and/or biosensor field-effect transistor - Bio-FET), which enable the evaluation of the biomarkers and/or ions that indicate the evolution of the process of the disease or the regeneration.

The referred electrochemical sensors (5, 6) use the technology of the ion-sensitive field-effect transistor - ISFET that allows measuring the concentration of ions, such as the H⁺, enabling the pH evaluation.

The piezoresistive, piezoelectric or capacitive sensors (7) are capable of identifying the strength of the injured tissue and/or the regenerated tissue.

The electric field sensors (8) present the technology of field electric transistors and/or resistometric and/or thermistors and allow the temperature evaluation.

The sensors that are based on the principle of photoplethysmography (9) allow evaluating the oxygen levels.

The electric stimulation electrode (10) and the magnetic stimulation subsystem (2) have direct communication with the three-dimensional model (19) and the surrounding tissue.

The electric stimulation triggers the cell activity, promoting the adhesion, proliferation, and cell differentiation, as well as the interaction with the three-dimensional magnetic model (19).

The magnetic stimulation, in its turn, activates the particles, also magnetic, incorporated in the three-dimensional models (19), in this manner releasing ions and therapeutic agents. Further, the magnetic activation of the particles stimulates the activity and cell interaction, promoting an endogenous environment which allows the triggering of the cascade of phenomena which will lead to the tissue regeneration.

The variation of the electric and magnetic parameters allows carrying out a customized treatment. In a preferred embodiment of the present invention, the electric stimulations can vary from 0.1 to 1 mA and the magnetic stimulations can vary in frequency (from 0.1 to 500 Hz) and in magnetic field (from 0.1 to 500 mT).

Apart from being able to generate a magnetic field, the intelligent biomimetic biodevice (18) of the invention is able to promote a hyperthermia effect. The increase in the temperature of the tissue is caused by the action of the magnetic stimulation coils (15) in the particles which, when excited, dissipate the heat and release the ions or the therapeutic agents.

The hyperthermia effect is quite interesting in the cases of tumor treatment, since cancer cells can remain after removal of the tumor. In these cases, the temperature is increased by the action of the magnetic field (reaching values from 40 to 46 °C), thus altering the physiology of the malignant cells, leading to apoptosis and cell necrosis.

The signal amplification subsystem (3) has the function of amplifying the information acquired by the several sensors by means of the RFID (16), wireless and / or Bluetooth (17) communication modules, allowing a real time monitoring of the therapy and the possibility of adapting the treatment. Depending on the type of power module (14) to be used, there can be a battery present.

The magnetic particles present in the three-dimensional model (19) are obtained from natural biocompatible, biodegradable and bioresorbable materials. In a preferred embodiment of the invention, the natural materials are selected from collagen, gelatin, and recombinant peptides, among others.

The particles further comprise several ions which grant biomimetic properties with the native tissues, whereby these are selected from one or more from calcium, iron, magnesium, carbonate, citrate, among others.

The particles are obtained from a biomineralization process, followed by emulsification or other additive manufacturing process, and can present sizes in nano or micro scale and be dense or porous.

The particles are bioactive and further comprise antimicrobial agents, such as strontium ions, zinc, silver, and therapeutic agents, such as drugs and growth factors, which properties vary according to the intended use.

Once obtained, the particles are incorporated in three-dimensional models (19) (for example, injectable models, hydrogels and/or scaffolds) by additive manufacturing technologies and then, applied to the body of the patient.

After being applied, the intelligent biomimetic biodevice (18) of the present invention is used in the prevention and treatment of diseases associated with the skeletal system, by means of the activation of the particles by means of the magnetic fields and the release of the therapeutic agents or ions contained therein.

In a preferred embodiment of the invention, the diseases associated with the skeletal system are selected from degenerative diseases, tumors, bone loss in case of need of dental implant and fractures, among others.

Further, the intelligent biomimetic biodevice (18) of the present invention is used in the monitoring of the evolution of the regeneration process by monitoring the treatment of specific indicators selected, such as strength, pH, oxygen, temperature and/or biomarkers.

The subject matter described above is provided as an illustration of the present invention and, therefore, cannot be interpreted so as to limit it. The terminology used herein with the purpose of describing preferred embodiments of the present invention, must not be interpreted to limit it to the same.

As used in the specification, the definite and indefinite articles, in their singular form, aim at the interpretation of also including the plural forms, unless the context of the description indicates, explicitly, the contrary. It will be understood that the expressions "comprise" and "include", when used in this description, specify the presence of the characteristics, the elements, the components, the steps, and the related operations, however, they do not exclude the possibility of other characteristics, elements, components, steps, and operations also being contemplated.

All the alterations, providing that they do not modify the essential characteristics of the claims that follow, must be considered as being within the scope of protection of the present invention.

### LIST OF REFERENCE INDICATIONS

- 1 -: Intelligent biomimetic surface
- 2 -: Magnetic stimulation subsystem
- 3 -: Signal amplification subsystem
- 4 -: Functional electronic subsystem
- 5 -: Electrochemical sensors for measuring the ions and/or biomarkers
- 6 -: Electrochemical sensors for pH measurement
- 7 -: Piezoresistive sensors for strength measurement
- 8 -: Electric field sensors for temperature measurement
- 9 -: Sensors that are based on the principle of photoplethysmography for oxygen measurement
- 10 -: Electric stimulation electrode
- 11 -: Processing module of the intelligent biomimetic surface transducers;
- 12 -: Central processing unit
- 13 -: Sensor signals amplification module
- 14 -: Power module
- 15 -: Magnetic stimulation coil
- 16 -: RFID communication module
- 17 -: WI-FI / BLUETOOTH communication module
- 18 -: Intelligent biomimetic biodevice
- 19 -: Three-dimensional model

### LIST OF CITATIONS

Follows the list of citations:

### PATENT LITERATURE

- US 2007/0238992 A1
- US 8,016,859 B2
- CN 101287408 A
- EP 2 510 873 B1
- CN 105943145 B
- US 2013/0138017 A1
- US 7,932,825 B2
- US 6,839,596 B
- US 7,024,249 B2

### NON-PATENTARY LITERATURE

- Patrício T. M. F., Mumcuogluv D., Montesi M., Panseri S., Witte-Bouma J., Garcia S. F., Sandri M., Tampieri A., Farrell E., Sprio S. (2021). Bio-inspired polymeric iron-doped hydroxyapatite microspheres as a tunable carrier of rhBMP-2, Materials Science and Engineering: C, Volume 119, 111410.
- Patrício T. M. F., Panseri S., Montesi M., Iafisco M., Sandri M., Tampieri A., Sprio S. (2019). Superparamagnetic hybrid microspheres affecting osteoblasts behavior, Materials Science and Engineering: C, 96, pp. 234-247, doi.org/10.1016/j.msec.2018.11.014.
- Patrício, T. M. F., Panseri, S., Sandri, M., Tampieri, A., & Sprio, S. (2017). New bioactive bone-like microspheres with intrinsic magnetic properties obtained by bio-inspired mineralisation process. Materials Science and Engineering: C, 77, 613-623, DOI: 10.1016/j .msec.2017.03.258.

## Claims

1. Intelligent biomimetic biodevice (18) **characterized by** comprising:
a) an intelligent biomimetic surface (1) comprising transducers, sensors (5, 6, 7, 8, 9) and electric stimulation electrode (10);
b) a magnetic stimulation subsystem (2) comprising at least two magnetic stimulation coils (15);
c) a signal amplification subsystem (3), comprising a signal amplification module (13) of the sensors (5, 6, 7, 8, 9), an RFID communication module (16) and a WI-FI / BLUETOOTH communication module (17); and
d) a functional electronic subsystem (4) comprising a transducer processing module (11) of the intelligent biomimetic surface (1); a power module (14) and a central processing unit (12);
wherein the referred intelligent biomimetic biodevice (18) is configured to communicate with a three-dimensional model (19) and activate particles incorporated in the same by means of electric and magnetic stimulation and presenting a Young modulus with tension that varies from 4.6 MPa to 20 MPa.

2. Intelligent biomimetic biodevice (18), according to claim 1, **characterized by** the intelligent biomimetic surface (1) being comprised by natural or synthetic biocompatible materials.

3. Intelligent biomimetic biodevice (18), according to any one of claims 1 or 2, **characterized by** the natural biocompatible materials being selected from one or more of the group consisting of collagen, gelatin, and recombinant peptides.

4. Intelligent biomimetic biodevice (18), according to any one of claims 1 or 2, **characterized by** the synthetic biocompatible materials being selected from one or more of the group consisting of polycaprolactone, polylactic acid, poly(lactic-co-glycolic acid).

5. Intelligent biomimetic biodevice (18), according to any one of the preceding claims, **characterized by** presenting a microneedle biomimetic geometry and/or a biomimetic geometry in the shape of membranes or suction pads.

6. Intelligent biomimetic biodevice (18), according to claim 5, **characterized by** the biomimetic geometry comprising enzymes or antibodies.

7. Intelligent biomimetic biodevice (18), according to any one of claims 5 or 6, **characterized by** the enzyme being a metalloproteinase enzyme.

8. Intelligent biomimetic biodevice (18), according to any one of claims 5 or 6, **characterized by** the antibodies being selected from one or more of osteonectin, osteocalcin, alkaline phosphatase, autophagy marker (LC3B) or interleukin-6 (IL-6).

9. Intelligent biomimetic biodevice (18), according to any one of the preceding claims, **characterized by** detecting analytes originating from bone tissue selected among treatment indicators and inflammation indicators.

10. Intelligent biomimetic biodevice (18), according to claim 9, **characterized by** the treatment indicators being selected from one or more of the group consisting in proteins, collagen, alkaline phosphatase, osteocalcin or calcium phosphate.

11. Intelligent biomimetic biodevice (18), according to claim 9, **characterized by** the inflammation indicators being selected from one or more of the group consisting in an expression of IL-6, the pH, the temperature, the oxygenation and the strength.

12. Intelligent biomimetic biodevice (18), according to any one of the preceding claims, **characterized by** the sensors (5, 6, 7, 8, 9) of the intelligent biomimetic surface (1) being selected from one or more of the group consisting in electrochemical sensors (5, 6), piezoresistive (piezoelectric or capacitive) (7), electric field sensors (8), and sensors that are based on the principle of photoplethysmography (9), or a combination thereof.

13. Intelligent biomimetic biodevice (18), according to any one of the preceding claims, **characterized by** the electrochemical sensors (5, 6) presenting potentiometry and/or amperometry mechanisms and/or voltammetry and/or biosensor field-effect transistor sensitive to ions and the electric field sensors presenting electric field transistor technology and/or resistometric and/or thermistors.

14. Intelligent biomimetic biodevice (18), according to any one of the preceding claims, **characterized by** the electric stimulation electrode (10) and the magnetic stimulation subsystem (2) having direct communication with the three-dimensional model (19) and the surrounding tissue.

15. Intelligent biomimetic biodevice (18), according to any one of the preceding claims, **characterized by** the electric stimulation varying from 0.1 to 1 mA.

16. Intelligent biomimetic biodevice (18), according to any one of the preceding claims, **characterized by** the magnetic stimulations varying in frequency from 0.1 to 500 Hz and in a magnetic field from 0.1 to 500 mT.

17. Intelligent biomimetic biodevice (18), according to any one of the preceding claims, **characterized by** the signal amplification subsystem (3) presenting a battery according to the power module (14) to be used.

18. Use of the intelligent biomimetic biodevice (18) as defined in claims 1 to 17, **characterized by** being in the activation of the particles by magnetic fields and release of the therapeutic agents or ions contained therein for the prevention and treatment of diseases associated with the skeletal system.

19. Use of the intelligent biomimetic biodevice (18) according to claim 18, **characterized by** the diseases associated with the skeletal system being selected from degenerative diseases, tumors, bone loss in case of need for dental implant and fractures, among others.

20. Use of the intelligent biomimetic biodevice (18) as defined in claims 1 to 17, **characterized by** being in the monitoring of the evolution of the regeneration process by means of the monitoring of the treatment of the treatment indicators and inflammation indicators.

21. Use of the intelligent biomimetic biodevice (18) according to claim 20, **characterized by** the treatment indicators being selected from one or more from the group consisting in proteins, collagen, alkaline phosphatase, osteocalcin, or calcium phosphate.

22. Use of the intelligent biomimetic biodevice (18) according to claim 20, **characterized by** the inflammation indicators being selected from the group consisting in the expression of interleukin-6 (Il-6), the pH, the temperature, the oxygenation, and the strength.
